Europäisches Patentamt

European Patent Office

Office européen des brevets

(19)

(11) Veröffentlichungsnummer : **0 066 287 B1**

## EUROPÄISCHE PATENTSCHRIFT
(12)

(45) Veröffentlichungstag der Patentschrift :
**10.09.86**

(21) Anmeldenummer : **82104779.2**

(22) Anmeldetag : **01.06.82**

(51) Int. Cl.⁴ : **C 01 G  1/00, C 01 G  1/06, C 07 C  9/02, C 07 C  5/00, C 07 C 45/00**

(54) Übergangsmetall-Komplexverbindungen und Verfahren zu ihrer Herstellung.

(30) Priorität : **03.06.81 DE 3121927**

(43) Veröffentlichungstag der Anmeldung :
**08.12.82 Patentblatt 82/49**

(45) Bekanntmachung des Hinweises auf die Patenterteilung : **10.09.86 Patentblatt 86/37**

(84) Benannte Vertragsstaaten :
**AT BE CH DE FR GB IT LI NL**

(56) Entgegenhaltungen :
**FR-A- 2 216 228**
**US-A- 3 933 919**
**CHEMISCHE BERICHTE, 114. Jahrgang, Heft 11, 3. November 1981, Seiten 3634-3642, Verlag Chemie GmbH, Weinheim, DE. G. SCHMID: "Au55(P(C6H5)3)12C16- ein Goldcluster ungewöhnlicher Grösse"**

(73) Patentinhaber : **WACKER-CHEMIE GMBH Prinzregentenstrasse 22 D-8000 München 22 (DE)**

(72) Erfinder : **Schmid, Günter, Prof. Dr. Laakmannsbusch 38 D-5620 Velbert 11 (Langenberg) (DE)**

**Beschreibung**

Die Erfindung betrifft Übergangsmetall-Komplexverbindungen, sogenannte Übergangsmetall-Cluster exzeptioneller Größe und Verfahren zu ihrer Herstellung.

Die größten bisher bekannten und isolierten Übergangsmetall-Cluster enthalten bis zu 38 Metallatome und tragen als Liganden vorwiegend Kohlenmonoxid, wie z. B. $(Rh_{15}(CO)_{27})^{3-}$ (S. Martinengo et al., J. Amer. Chem. Soc. *100*, 7096 (1978)) ; $(Rh_{17}(CO)_{32}S_2)^{3-}$ (J. Vidal et al., Inorg. Chem. *17*, 2574 (1978)) ; $(Rh_{22}(CO)_{37})^{4-}$ (S. Martinengo et al., J. Amer. Chem. Soc. *102*, 7564 (1980)) ; $(Pt_{19}(CO)_{22})^{4-}$ (D. M. Washechek et al., J. Amer. Chem. Soc. *101*, 6110 (1979)).

Goldcluster der Formel $Au_{11}L_7X_3$ und $(Au_{13}(LL)_6)^{4+}$ (L = Phosphan, LL = Diphosphan, X = Halogen, Pseudohalogen (F. Cariati und L. Naldini, Inorg. Chim. Acta *5*, 1972 (1971), P. L. Ballon. M. Manassero und M. Sansoni, J. Chem. Soc., Dalton *1972*, 1481)) enthalten erstmals mehr Metallatome als Liganden. Die 10 bzw. 12 peripheren Goldatome bilden nur je einen terminalen Phosphan- bzw. Halogen- oder Pseudohalogenliganden. Das elfte (dreizehnte) Goldatom befindet sich im Zentrum eines unvollständigen (vollständigen) Ikosaeders. Bei Clustern, die sich aus dichtesten Kugelpackungen aufbauen, wird der Anteil der Oberflächenatome mit zunehmender Gesamtzahl der Metallatome stetig kleiner. So kann man berechnen, daß der Anteil der Oberflächenatome von 100 % bei 4 oder 6 Atomen, 92 % bei 13 Atomen (kleinstes mögliches Kuboktaeder) über 52 % bei 309 Atomen bis auf 15 % bei 21 127 Atomen abnimmt.

Vom Standpunkt der Katalyse her erscheint es wünschenswert, solche Cluster herzustellen, die in ihren Eigenschaften zwischen den bekannten Komplexkatalysatoren mit einem oder wenigen (maximal 38) Metallatomen und den rein metallischen Katalysatoren stehen. Synthesen zu solchen Stoffen sind bisher nicht bekannt.

Der Erfindung lag deshalb die Aufgabe zugrunde, derartige Verbindungen aufzufinden und einen Syntheseweg zu ihrer Herstellung anzugeben.

Gelöst wurde die Aufgabe durch Übergangsmetall-Komplexverbindungen der allgemeinen Formel $M_{55}L_{12}X_p$, wobei M ein Übergangsmetall der I., V., VI., VII. oder VIII. Nebengruppe des periodischen Systems der Elemente nach Mendeljeff (vgl. Handbook of Chemistry and Physics, 55th Edition 1974-1975, CRC-Press, abgedruckt in der Innenseite der Einbanddecke) bedeutet, L für Liganden mit Elektronendonatoreigenschaften steht, X Halogen ist und p gleich 6 oder 20.

Herstellen lassen sich diese neuen Verbindungen erfindungsgemäß durch ein Verfahren, welches dadurch gekennzeichnet ist, daß man Komplexverbindungen der allgemeinen Formel $L_nMX_m$, wobei M, L und X die oben angegebene Bedeutung haben und n eine Zahl von 1 bis 5 sowie m eine Zahl von 1 bis 4 bedeuten, mit Bor- oder Aluminiumwasserstoffen umsetzt.

Unter Liganden mit Elektronendonatoreigenschaften werden allgemein Moleküle verstanden, die über π-Elektronenpaare oder freie Elektronenpaare verfügen, wie beispielsweise Kohlenmonoxid, Amine, Phosphane, Diphosphane, Arsane, Diarsane, Phosphite, Stibine, Stannan etc. Die Größe dieser Liganden im Verhältnis zum Atomradius des Metallatoms in diesen neuen Übergangsmetall-Komplexen spielt dabei eine gewisse Rolle hinsichtlich der Stabilität dieser neuen Übergangsmetall-Komplexverbindungen. Beispielsweise ist ein Rhodium-Cluster mit Tritertiärbutylphosphin stabiler als ein Rhodium-Cluster mit Trimethylphosphit als Ligand, während bei Komplexverbindungen mit dem kleineren Nickel der Fall gerade umgekehrt liegt.

Gut herstellbar sind beispielsweise Verbindungen des Goldes, Vanadiums, Chroms, Molybdäns, Mangans, Kobalts, Nickels, Rutheniums, Rhodiums oder Palladiums.

Die Umsetzung der vorstehend definierten Komplexverbindungen $L_nMX_m$ zu den erfindungsgemäßen neuen Übergangsmetall-Komplexverbindungen erfolgt unter Schutzgas, wie beispielsweise Argon oder Stickstoff und zweckmäßig in einem Lösungsmittel, vorzugsweise in Aromaten, wie beispielsweise Benzol, Toluol oder Pyridin, gut geeignet sind aber auch Methylenchlorid oder Ether, wie insbesondere Tetrahydrofuran. Die Aluminium- und Borwasserstoffe werden in die Lösung der Komplexverbindungen $L_nMX_m$ eingebracht, wobei man zweckmäßig bei Temperaturen zwischen Raumtemperatur und der Siedetemperatur des jeweilig eingesetzten Lösungsmittels arbeitet, also etwa in einem Temperaturbereich von 20 bis 120 °C. Die neuen Verbindungen fallen zum Teil unmittelbar als feinkristalliner Niederschlag aus und können durch Abfiltrieren abgetrennt werden. Häufiger ist es aber zweckmäßig, das Lösungsmittel unter reduziertem Druck schonend weitgehend abzudestillieren und den verbleibenden mehr oder minder intensiv gefärbten öligen Rückstand mit einem unpolaren Lösungsmittel, beispielsweise Petrolether, zu versetzen und den sich bildenden Feststoff in einem geeigneten Lösungsmittel umzufällen oder umzukristallisieren und abzutrennen. Die Umsetzung kann prinzipiell in kontinuierlicher oder diskontinuierlicher Arbeitsweise erfolgen.

Die neuen Übergangsmetall-Cluster sind hochaktive Katalysatoren bei der katalytischen Hydrierung von beispielsweise C=C-Doppelbindungen oder C≡C-Dreifachbindungen, Carbonylen, Nitrilen und Isonitrilen oder bei der katalytischen Reduktion der $NO_2$-Funktion zu Aminen. In der Hydroformylierungsreaktion, bei der Wassergassynthese, bei der Isomerisierung und Cyclisierung sowie bei der Reduktion von Kohlenmonoxid mit Wasserstoff zu Kohlenwasserstoffen, Alkoholen oder Aldehyden sind sie ebenfalls katalytisch wirksam. Darüberhinaus lassen sich die neuen Übergangsmetall-Komplexverbindungen in vorzüglicher Weise zur Metallbeschichtung beliebiger Oberflächen einsetzen.

Hierzu wird der zu beschichtende sorgfältig gereinigte Gegenstand in eine Lösung der jeweiligen Komplexverbindung eingetaucht und ggf. erwärmt. Die Dauer des Eintauchens, die Höhe der gewählten Temperatur sowie die Stabilität des jeweilig eingesetzten erfindungsgemäßen Übergangsmetall-Komplexes sowie seine Konzentration in der Lösung sind dabei für die in der Zeiteinheit erzielbare Schichtdicke maßgeblich.

## Beispiel 1

Darstellung von $Au_{55}(P(C_6H_5)_3)_{12}Cl_6$

Ein 250 ml Dreihalskolben, versehen mit Rührer, Innenthermometer, Gaseinleitungsrohr und Rückflußkühler wird unter Argon als Schutzgas mit 3,94 g $(C_6H_5)_3PAuCl$ (7,9 mMol) und 150 ml wasserfreiem Benzol beschickt. Unter Rühren leitet man einen mäßigen Strom an Diboran durch die Lösung, die sich sofort violett, später dunkelbraun, färbt. Während des Einleitens steigert man die Temperatur im Reaktionsgefäß auf 50 °C. Nach 30 bis 60 Minuten fällt ein dunkler Niederschlag aus, während die überstehende Lösung nahezu farblos ist. Der Niederschlag wird über eine Umkehrfritte abfiltriert und in möglichst wenig Methylenchlorid gelöst. Dabei bildet sich eine dunkelrot-braune Lösung, aus der nach dem erneuten Abfritten durch Versetzen mit Petrolether ein dunkelbrauner Stoff ausgefällt wird. Zur weiteren Reinigung wird erneut in Methylenchlorid gelöst und über eine 4 bis 5 cm dicke Schicht Kieselgur filtriert, um eventuell anhaftende Reste kolloidalen Goldes abzutrennen. Nach erneutem Ausfällen erhält man 0,8 g $Au_{55}(P(C_6H_5)_3)_{12}Cl_6$ (39,0 %, bezogen auf eingesetztes Triphenylphosphingoldchlorid).

Aus dem Filtrat der Reaktionslösung fällt im Verlauf mehrerer Tage ein weiterer braunschwarzer Niederschlag aus, der noch nicht charakterisiert werden konnte. Nach Abfiltrieren dieses Niederschlages läßt sich aus der Lösung durch Zugabe von Petrolether $(C_6H_5)_3P—BH_3$ isolieren und durch IR-Spektrenvergleich mit authentischen Proben sowie durch Elementaranalyse charakterisieren.

Analyse der Gold-Komplexverbindung :

Berechnet : für $Au_{55}(P(C_6H_5)_3)_{12}Cl_6$
       C 18,28  H 1,28  Au 76,33  Cl 1,50  P 2,62
Gefunden :  C 17,66  H 1,28  Au 76,10  Cl 1,70  P 2,60

$C_{18}H_{18}BP$ (276,0) Berechnet :  C 78,26  H 6,52
                  Gefunden :  C 77,22  H 6,54
Schmelzpunkt : 180 °C (Lit. 185 °C)

Thermolyse von $Au_{55}(P(C_6H_5)_3)_{12}Cl_6$ :

200 mg (0,085 mMol) $Au_{55}(P(C_6H_5)_3)_{12}Cl_6$ werden in 20 ml Pyridin gelöst und 3 Tage lang auf 50 °C erwärmt. Dabei scheidet sich metallisches Gold teils als Spiegel, teils in feinverteilter, dunkler Form ab, während die Lösung farblos wird. Die Auswaage an Gold beträgt 130 mg (berechnet : 137 mg).

Struktur der Gold-Komplexverbindung :

Die Gold-Komplexverbindung $Au_{55}(P(C_6H_5)_3)_{12}Cl_6$ zeigt im Mössbauer-Spektrum vier unterschiedliche Sorten von Goldatomen, einen metallischen Goldkern, durch $P(C_6H_5)_3$- bzw. Cl-Liganden koordinierte Goldatome sowie unkoordiniertes Oberflächengold. Das IR-Spektrum zeigt eine Verschiebung der Gold-Chlor-Schwingung von 330 $cm^{-1}$ $((C_6H_5)_3PAuCl)$ nach 280 $cm^{-1}$ in der neuen Komplexverbindung. Das Molekulargewicht wurde aus dem Sedimentationskoeffizienten zu 15 760 (berechnet 14195) bestimmt. Die osmometrische Bestimmung ergab eine Molekularmasse von 13 000.

## Beispiel 2

Darstellung von $Rh_{55}(P(C_4H_9)_3^i)_{12}Cl_{20}$

Ein 250 ml Dreihalskolben, versehen mit Rührer, Innenthermometer, Gaseinleitrohr und Rückflußkühler wird unter Argon als Schutzgas mit 2,0 g $(P(C_4H_9)_3^i)_2RhCl$ in 100 ml wasserfreiem Benzol beschickt. Man leitet einen gleichmäßigen Strom an Diboran in die Lösung und erwärmt gleichzeitig auf 50 bis 60 °C. Nach etwa 30 Minuten hat sich eine dunkelbraune Lösung gebildet, welche nach dem Abkühlen von etwas Ungelöstem abgetrennt wird. Man entfernt das Lösungsmittel im Vakuum und versetzt den dunklen, öligen Rückstand mit Petrolether. Nach mehrstündigem Rühren bildet sich ein dunkelbrauner Feststoff, der von der bräunlichen Lösung abfiltriert wird. Durch Lösen des Feststoffes in Aceton oder Chloroform und anschließendem Ausfällen mittels Petrolether wird die Verbindung gereinigt.

Ausbeute, bezogen auf eingesetztes $((C_4H_9)_3^iP)_2RhCl$ = 75 % der Theorie.

Beispiel 3

Darstellung von $Ru_{55}(P(C_4H_9)_3^t)_{12}Cl_{20}$

0,5 g $RuCl_3 2H_2O$, 2,0 ml $(C_4H_9)_3^t P$ und 60 ml Tetrahydrofuran werden in einem 250 ml-Dreihalskolben, versehen mit Rührer, Gaseinleitungsrohr und Stickstoffüberleitung vorgelegt und mit einem gleichmäßigen Gasstrom von Diboran zur Reaktion gebracht. Die Lösung färbt sich im Verlauf von 30 bis 60 Minuten dunkelbraun. Man entfernt anschließend das Lösungsmittel im Vakuum und behandelt das zurückbleibende Öl mit Petrolether, wobei es sich verfestigt. Man nimmt das braun-schwarze Produkt in Aceton auf, filtriert von Ungelöstem ab und fällt aus der Acetonlösung schwarzes pyrophores $Ru_{55}(P(C_4H_9)_3^t)_{12}Cl_{20}$ mit Petrolether aus. Ausbeute 60 %, bezogen auf eingesetztes Ruthenium.

Beispiel 4

Darstellung von $Pd_{55}(P(C_4H_9)_3^t)_{12}Cl_{20}$

3,2 g $(P(C_4H_9)_3^t)_2PdCl_2$ werden in 150 ml Toluol gelöst und in einem 250 ml-Dreihalskolben, versehen mit Rückflußkühler, Gaseinleitungsrohr und Stickstoffüberleitung, Innenthermometer und Rührer, 40 Minuten bei 70 bis 80 °C mit einem gleichmäßigen Gasstrom von Diboran umgesetzt. Die entstandene dunkelbraune Lösung wird nach dem Erkalten abgefrittet und vom Lösungsmittel befreit. Den Rückstand wäscht man mehrmals mit Diethylether und trocknet anschließend im Vakuum. Ausbeute 48 % der Theorie, bezogen auf eingesetztes Palladium.

Beispiel 5

Darstellung von $Ni_{55}(P(OCH_3)_3)_{12}Cl_{20}$

In einem 250 ml-Dreihalskolben mit Rückflußkühler, Gaseinleitungsrohr und Stickstoffüberleitung, Thermometer und Magnetrührer werden 2,0 g wasserfreies Nickelchlorid und 4,0 ml $P(OCH_3)_3$ in 80 ml Tetrahydrofuran bei 60 °C mit gasförmigem Diboran unter sorgfältigstem Luftausschluß umgesetzt. Die Lösung färbt sich allmählich dunkel, während ein braunschwarzer Niederschlag ausfällt. Man frittet ab, wäscht den Niederschlag mit Benzol und trocknet ihn im Vakuum. Ausbeute 90 % pyrophores $Ni_{55}(P(OCH_3)_3)_{12}Cl_{20}$, bezogen auf eingesetztes Nickel.

Beispiel 6

Darstellung von $Co_{55}(P(OCH_3)_3)_{12}Cl_{20}$

Ein 250 ml-Dreihalskolben, versehen mit Rückflußkühler, Gaseinleitungsrohr, Thermometer und Magnetrührer wird unter Stickstoff mit 2,0 g wasserfreiem $CoCl_2$, 4,0 ml $P(OCH_3)_3$ und 80 ml Tetrahydrofuran beschickt. Man erwärmt auf 60 °C und leitet einen gleichmäßigen Diboranstrom durch die Lösung. Es scheidet sich ein braunschwarzer Niederschlag aus $Co_{55}(P(OCH_3)_3)_{12}Cl_{20}$ ab, der abgefrittet, mehrmals mit Benzol gewaschen und im Vakuum getrocknet wird. Ausbeute 70 % der Theorie.

Beispiel 7

Hydrierung von Hexen-1

50 ml Hexen-1, in Tetrahydrofuran gelöst, werden in einem Rührautoklaven in Gegenwart von 50 ml $Rh_{55}(P(C_4H_9)_3^t)_{12}Cl_{20}$ mit Wasserstoff bei 70 atm Druck und Raumtemperatur (20 °C) umgesetzt. Innerhalb von 15 bis 20 Minuten findet quantitative Hydrierung zu Hexan statt.

Beispiel 8

Hydrierung von Penten-1

Es wurde entsprechend Beispiel 7 verfahren und 99,7 Vol-% n-Pentan erhalten.

Beispiel 9

Hydrierung von Hexin-3

Es wurde entsprechend Beispiel 7 verfahren (allerdings bei Normaldruck von 1 atm) und 100 Vol-% n-Hexen erhalten.

4

## Beispiel 10

Hydroformylierung von Hexen-1

Hexen-1 wird, in Tetrahydrofuran gelöst, in einem Rührautoklaven in Gegenwart von 50 mg $Rh_{55}(P(C_4H_9)_3^t)_{12}Cl_{20}$ bei 130 atm mit einem 1 : 1 Gemisch von Wasserstoff und Kohlenmonoxid bei Raumtemperatur (20 °C) umgesetzt. Nach 12-stündiger Reaktionszeit lassen sich in der Lösung gaschromatographisch 25 Vol-% Heptanal als Isomerengemisch nachweisen.

## Beispiel 11

Hydroformylierung von Cyclopenten

Es wurde entsprechend Beispiel 10 Cyclopenten umgesetzt und 22 Vol-% Cyclopentancarbaldehyd erhalten.

## Beispiel 12

Hydroformylierung von Cyclohexen

Es wurde entsprechend Beispiel 10 Cyclohexen umgesetzt (allerdings bei einem Druck von 150 atm) und 30 Vol-% Cyclohexancarbaldehyd erhalten.

## Beispiel 13

Hydrierung von Penten-1

25 ml Penten-1 werden mit einer Lösung von 50 mg $Ru_{55}(P(C_4H_9)_3^t)_{12}Cl_{20}$ in 2 ml Diacetonalkohol versetzt. Man leitet 1 1/2 Stunden Wasserstoff bei Raumtemperatur (20 °C) und Normaldruck (1 atm) durch die Lösung. Gaschromatographisch läßt sich nach dieser Zeit nur noch n-Pentan nachweisen.

## Beispiel 14

Hydroformylierung von Hexen-1

Eine Lösung von 50 mg $Ru_{55}(P(C_4H_9)_3^t)_{12}Cl_{20}$ in 50 ml Tetrahydrofuran wird mit 50 ml Hexen-1 versetzt. In einem Autoklaven werden 75 atm Kohlenmonoxid sowie 75 atm Wasserstoff aufgedrückt. Nach 10-stündiger Reaktionszeit lassen sich gaschromatographisch 25 Vol-% Heptanal als Isomerengemisch nachweisen.

## Beispiel 15

Goldbeschichtung

Die zu vergoldenden Gegenstände aus Glas, Kunststoff und Metall werden bei Raumtemperatur (20 °C) in eine Lösung von $Au_{55}(P(C_6H_5)_3)_{12}Cl_6$ in Methylenchlorid getaucht. Die Vergoldung tritt von selbst ein, Temperaturerhöhung beschleunigt den Vorgang. Die Schichtdicke hängt von der Dauer des Vorgangs sowie der Konzentration der Lösung ab. Die Goldoberflächen besitzen auch in dünnster Schicht elektrische Leitfähigkeit.

## Beispiel 16

Goldbeschichtung von dispersen Materialien

Disperse Materialien werden mit beliebig dünnen Goldschichten belegt, indem das Material, wie z. B. hochdisperse Kieselsäure oder Aluminiumoxid, in einer Lösung von $Au_{55}(P(C_6H_5)_3)_{12}Cl_6$ in Methylenchlorid getränkt und danach abfiltriert und getrocknet wird. Anschließendes Erwärmen des Materials auf Temperaturen über 100 °C führt zur Zersetzung des Komplexes am Material, wobei kolloidales Gold abgeschieden wird. Nachfolgendes Abspülen mit Methylenchlorid entfernt anderes, bei der Zersetzung gebildetes Material.

**Patentansprüche**

1. Übergangsmetall-Komplexverbindungen der allgemeinen Formel $M_{55}L_{12}X_p$, wobei M ein Über-

gangsmetall der I., V., VI., VII. oder VIII. Nebengruppe des periodischen Systems der Elemente nach Mendelejeff bedeutet, L für Liganden mit Elektronendonatoreigenschaften steht, X Halogen ist und p gleich 6 oder 20.

2. Verfahren zur Herstellung von Übergangsmetall-Komplexverbindungen nach Anspruch 1, dadurch gekennzeichnet, daß man Komplexverbindungen der allgemeinen Formel $L_nMX_m$, wobei M, L und X die in Anspruch 1 angegebene Bedeutung haben und n eine Zahl von 1 bis 5 sowie m eine Zahl von 1 bis 4 bedeuten, mit Bor- oder Aluminiumwasserstoffen umsetzt.

## Claims

1. Transition metal complex compounds of the general formula $M_{55}L_{12}X_p$ in which m represents a transition metal of sub-group I, V, VI, VII or VIII of the Mendeleev Periodic Table of the Elements, L represents a ligand having electron donor properties, X is halogen and p equals 6 or 20.

2. Process for the manufacture of transition metal complex compounds according to claim 1, characterised in that complex compounds of the general formula $L_nMX_m$ in which M, L and X have the meanings given in claim 1 and n represents a number from 1 to 5 and m represents a number from 1 to 4, are reacted with boron hydride or aluminium hydride.

## Revendications

1. Complexes de métaux de transition répondant à la formule générale $M_{55}L_{12}X_p$ dans laquelle M représente un métal de transition appartenant à l'un des groupes secondaires I, V, VI, VII et VIII de la classification périodique des éléments selon Mendeléeff, L représente un coordinat ayant des propriétés de donneur d'électrons, X représente un halogène et p un nombre égal à 6 ou à 20.

2. Procédé de préparation de complexes de métaux de transition selon la revendication 1, procédé caractérisé en ce qu'on fait réagir des complexes répondant à la formule générale $L_nMX_m$ dans laquelle M, L et X ont les significations données à la revendication 1, n désigne un nombre de 1 à 5 et m un nombre de 1 à 4, avec un hydrure de bore ou d'aluminium.